Europäisches Patentamt

European Patent Office

Office européen des brevets

(11) Publication number: **0 292 970**
**A1**

## (12) EUROPEAN PATENT APPLICATION

(21) Application number: **88108427.1**

(22) Date of filing: **26.05.88**

(51) Int. Cl.⁴: **A41B 13/02 , C09J 7/04**

(30) Priority: **26.05.87 JP 79472/87**
**26.05.87 JP 79749/87**

(43) Date of publication of application:
**30.11.88 Bulletin 88/48**

(84) Designated Contracting States:
**DE FR IT SE**

(71) Applicant: **UNI-CHARM CORPORATION**
**182, Shimobun Kinsei-cho**
**Kawanoe-shi Ehime-ken(JP)**

(72) Inventor: **Suzuki, Migaku**
**221-11, Shimobun Kinsei-cho**
**Kawanoe-shi Ehime-ken(JP)**
Inventor: **Ochi, Mitsuzo**
**4-172 Ohaza-Fujiwara Doi-cho**
**Uma-gun Ehime-ken(JP)**
Inventor: **Kudo, Takeshi**
**3389-11 Kawanoe-cho Kawanoe-shi**
**Ehime-ken(JP)**

(74) Representative: **Staeger, Sigurd, Dipl.-Ing. et**
**al**
**Patentanwälte Dipl.-Ing. S. Staeger Dipl.-Ing.**
**Dipl.-Wirtsch.-Ing. R. Sperling Müllerstrasse**
**31**
**D-8000 München 5(DE)**

(54) Fastening tape for disposable absorbent article.

(57) The disclosure describes a fastening tape for disposable absorbent articles such as diapers comprising an elongated base strip (16) having a fixed end (17) and a free end (18). The free end (18) is superimposed over the fixed end (17). The fixed end (17) includes adhesive coatings (20) on its underside which are adhesively applied onto a topsheet of the diaper (10) and release coatings (21) on its upperside. The free end (18) includes adhesive coatings (22), and the coatings (22) are received on the release coatings (21).

FIG.1

# FASTENING TAPE FOR DISPOSABLE ABSORBENT ARTICLE

## BACKGROUND OF THE INVENTION

The present invention relates to a pressure-sensitive adhesive fastening tape for disposable absorbent articles which are applied to a backwaist portion of a disposable absorbent article for affixing the article around a wearer.

In recent years, disposable absorbent articles have been widely accepted in the market place since they may be discarded after single use and need not be laundered. Typical absorbent articles comprise a fluid-pervious topsheet generally of a non-woven fabric, a liquid-impervious backsheet generally of plastic film, and an absorbent layer interposed between the topsheet and backsheet. The diapers are also provided with pressure-sensitive adhesive fastening tapes for connecting front and backwaist portions of the article. Prior fastening tapes include a fastening strip having free and fixed ends. An innerface of the fixed end is to be bonded to the backwaist portion of the article backsheet. Adhesive coatings are provided on an inner surface of the free end for pressing onto a front waist portion of the article backsheet. A release sheet is provided on a backwaist portion of the article topsheet to receive the free end and protect the adhesive coatings. Since such construction requires the two tape ends to be placed on opposite faces of the article, a folding operation therefor must be incorporated by the article manufacturers which decrease a production rate.

## SUMMARY OF THE INVENTION

A principle object of the present invention is the provision of a fastening tape for disposable absorbent articles so improved that the problems encountered by the prior invention as mentioned above may be solved.

Other objects and advantages of the present invention will be apparent from reading the following description.

According to the present invention there is provided a pressure-sensitive fastening tape for disposable absorbent article comprising: an elongated base strip; a fixed end and a free end divided by a fold of said base strip, said free end being superimposed over said fixed end; and said fixed end including first adhesive coatings on its underside and release coatings on its upperside, said release coatings receiving second adhesive coatings provided on an underside of said free end.

In a preferred embodiment of the present invention, said fastening tape is formed of a self-wound tape stock roll, said roll comprising longitudinally coextensive first and second strips defined by a fold of a continuous base strip, said second strip carrying a continuous band of adhesive coatings on its upperside and a continuous band of release coatings on its underside which receives a continuous band of adhesive coatings provided on an upperside of the first strip, said base strip having a multiplicity of intermittent slit lines which extends across the whole width thereof for defining individual fastening tapes in a juxaposed relationship.

The fastening tape may be applied to the disposable absorbent article by merely attaching aid first coatings onto a topsheet thereof, and requires no release tape separately formed from itself.

## DESCRIPTION OF THE DRAWINGS

Fig. 1 is a plan view of a disposable diaper having a pressure-sensitive adhesive fastening tape of this invention.

Fig. 2 is a sectional view along line 2 - 2 of Fig.l.

Fig. 3 is a plan view of the fastening tape as developed.

Fig. 4 is a longitudinal sectional view of the fastening tape.

Fig. 5 is a sectional view of a tape base strip.

Fig. 6 is a perspective view of a rolled tape stock in which individual fastening tape is seperated for use.

## DETAILED DESCRIPTION OF THE INVENTION

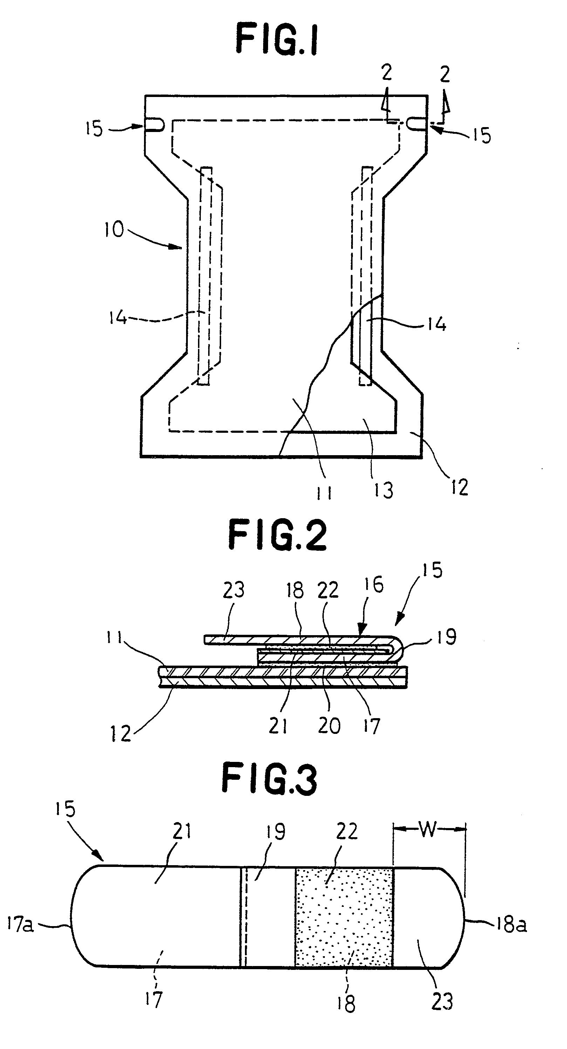

In Fig. 1, there is shown a preferred embodiment of the present invention as applied to a disposable diaper. The disposable diaper 10 comprises a liquid-permeable topsheet 11 generally of a non-woven fabric, a liquid-impermeable backsheet 12 generally of a plastic film and an absorbent core 13 interposed between the top and back sheets 11 and 12. The absorbent core 13, may be manufactured from comminuted wood pulp. The topsheet 11 is coextensive with and superposed on the backsheet 12 thereby to form a side flap on each longitudinal side of the diaper 10. An elastic member 14 is attached in each side flap to provide elasticity thereto. An adhesive fastening tape 15 is applied to each lateral end of a diaper backwaist portion.

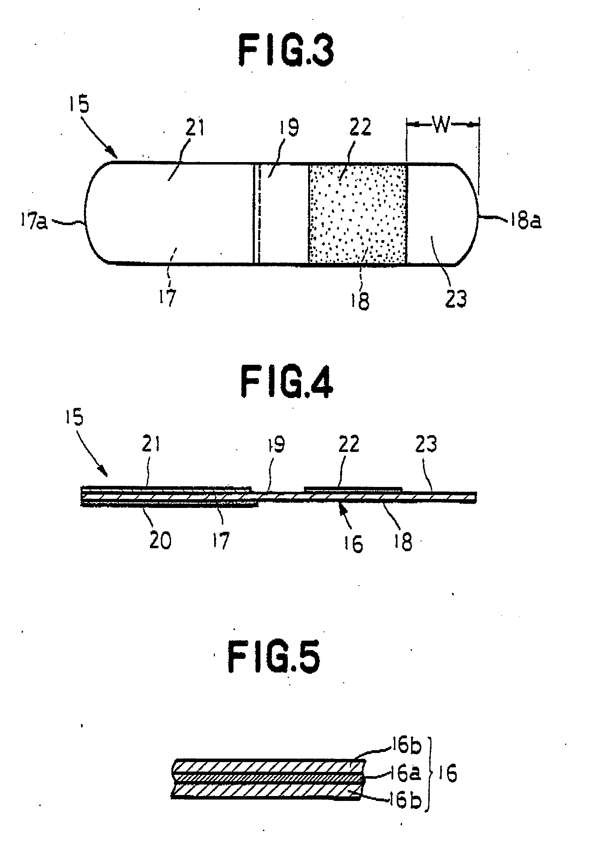

As shown in Figs. 2 to 4, the fastening tape 15 includes a flat base strip 16. The strip 16 is divided into a fixed end 17 and a free end 18 by a fold 19. The fixed end 17 has adhesive coatings 20 on its underside which is adhesively applied onto the topsheet 11. Suitable release coatings 21 are also provided on an upperside of the fixed end 17. The free end 18 is superimposed over the fixed end 17 and includes adhesive coatings 22 on its innerside which are received by the release coatings 21 of the fixed end 17. The fold 19 preferably stays inboard of the lateral edge of the diaper 10. The adhesive coatings 22 stop short of the terminal edge of the free end 18 to define a non-adhesive portion 23 for finger grip.

The fixed end 17 has a round or curvilinear terminal edge 17a which preferably describes a circular arc of 5 to 50mm in radius. The curvilinear terminal edge 17a provides a suitable protection against any damage which corner edges might cause to infant's skin when placed in contact therewith. Instead, the fixed end 17 may be cut round only at its terminal corner edges. The free end 18 also has a round or curvilinear terminal edge 18a which is intended to protect mother's fingers when gripping the free end 18 prior to a fastening procedure. The non-adhesive portion 23 provides a suitable finger grip means and preferably extends beyond the terminal edge of the fixed end 17. A suitable length W of the portion 23 may be 2 to 40mm for this purpose. When another adhesive sheet is additionally sandwiched between the free and fixed ends 18,17 to provide a resealable property, such sheet may also be cut round at its free terminal edge and similarly include a non-adhesive portion.

The base strip 16 may be any flexible material and one preferred material is shown in Fig. 5. This base strip 16 comprises a non-woven fabric layer 16a sandwiched between plastic layers 16b,16b. These layers may be attached together by any conventional means such as heat-sealing or adhesive-lamination.

The attachment is preferably performed in a patterned configuration, i.e., in dotted or stripe adhesive pattern, more preferably in a heat-embossed pattern so as to define non-secured free zones which effectively contribute to flexibility, soft touch and good apperance of the base strip 16. The non-woven fabric layer 16a preferably has a basis weight range of 7 to 100g/m2 and comprises a wide variety of fibers, i.e., natural fibers or synthetic fibers. A preferred example may be a spunbonded polypropylene. Each plastic layer 16b is

preferably 15 to 35$\mu$m thick and a suitable material therefor may be a low density polyethylene film having a density range of 0.915 to 0.925/cm$^3$ which provides the strip 16 with drapability and self-bearancy. Other suitable plastic materials are polyamide, polyester, polyolefines such as polypropylene and polyvinyl-chrolide. Suitable release coatings 21 may be any materials known in the art, such as silicones, fluorines, long-chain alkyls.

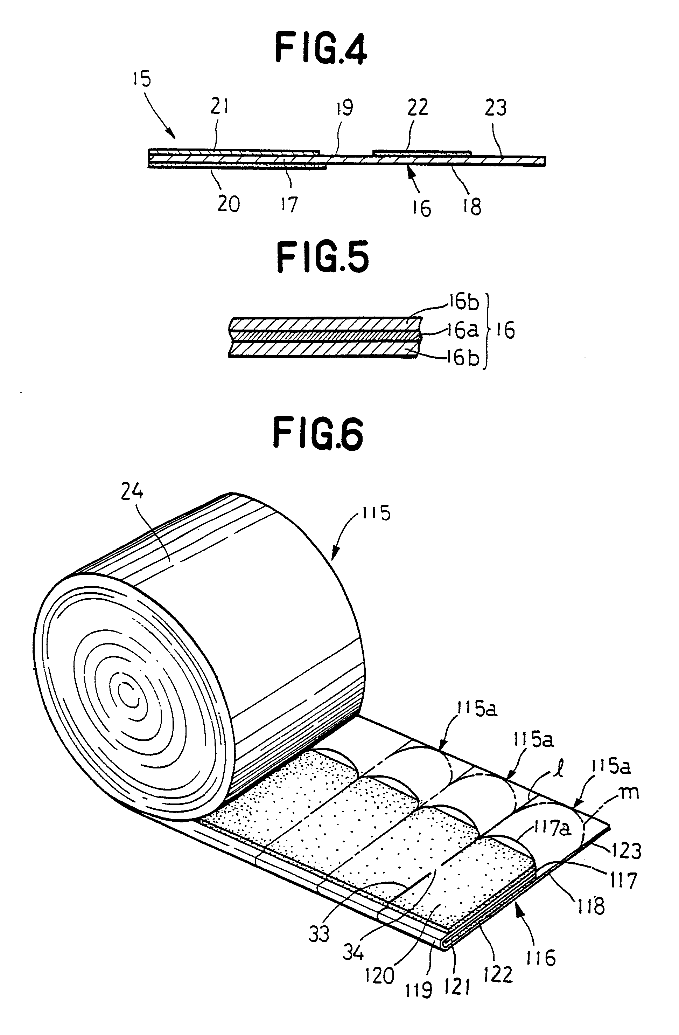

The fastening tape construction so described and shown in Fig. 2 may be more preferably supplied in the form of a self-wound tape stock roll 115, as seen in Fig. 6. For this purpose additional release coatings 24 may be provided on an underside of the free end 18 on which the adhesive coatings 20 are received when the tape stock is self-wound.

Fig. 6 shows the preferred tape stock roll 115 which comprises longitudinally coextensive first strip 118 and second strip 117 defined by a fold 127 of a continuous base strip 116. The first strip 118 has a longitudinal band of adhesive coatings 122 on its upperside which are received by a longitudinally-extending continuous band of release coatings 121 provided on a underside of the second strip 117. Another continuous band of adhesive coatings 120 is provided on an upperside of the second strip 117. The first and second strip 118, 117 respectively provide the free end 18 and the fixed end 17 of the foregoing fastening tape 15. The first strip 118 has a wider transverse length than that of the second strip 117 to provide a wider non-adhesive band 123 which corresponds to the non-adhesive portion 23 of the free end 18. From the foregoing, it is clear that the first and second strips 118 and 117 may have the same width as desired.

A multiplicity of intermittent slit line $\mathcal{l}$ is provided transversely of the base strip 116 at a longitudinally spaced interval. Each intermittent slit line $\mathcal{l}$ extends across the whole width of the base strip 116 and forms pairs of a slit portion 33 and a non-slit portion 34 in a repeated patters. The slit portions 33 penetrate both the first and second strip 118, 117. The non-slit portion 34 has a preferable width of 0.3 to 3.0mm. As apparent from the drawing, a series of individual fastening tape correspondent 115a are longitudinally juxtaposed by the intermittent slit lines $\mathcal{l}$. The opposite terminal portions of the line $\mathcal{l}$ may preferably be occupied by the slit portions 33 for easy separation of the individual fastening tape correspondent 115a from the tape stock roll 15.

At least one lateral edge of the continuous base strip 116 may be cut along a curved line 117a to provide the tape correspondent 115a with at least one round or curvilinear terminal edge. Alternatively, a curved intermittent slit line m may be provided along at least one lateral edge of the base strip 116 for easy separation as shown in Fig. 6.

In use, the tape stock roll 115 is unrolled and the individual tape correspondent 115a is seriously separated therefrom along the intermittent slit lines ι , for example, under a vacuum influence. The individual tape correspondent 115a is then transferred and placed under pressure onto a determined area of the diaper topsheet 11 for attachment.

## Claims

(1) A pressure-sensitive fastening tape for disposable absorbent article comprising:

an elongated base strip;

a fixed end and a free end divided by a fold of said base strip, said free end being superimposed over said fixed end; and

said fixed end including first adhesive coatings on its underside and release coatings on its upperside, said release coatings receiving second adhesive coatings provided on an underside of said free end.

(2) The fastening tape of Claim (1), wherein said second adhesive coatings stop short of a terminal edge of the free end to define a non-adhesive portion for finger grip.

(3) The fastening tape of Claim (2) wherein said terminal edge of the free end is configured curvilinear.

(4) The fastening tape of Claim (3) wherein said curvilinear terminal edge of the free end describes a circular arc of 5 to 50mm in radius.

(5) The fastening tape of Claim (2) wherein said non-adhesive portion extends beyond a terminal edge of the fixed end.

(6) The fastening tape of Claim (2) wherein said non-adhesive portion has a width range of 2 to 40mm.

(7) The fastening tape of Claim (1) wherein said fixed end has a curvilinear terminal edge.

(8) The fastening tape of Claim (1) wherein said base strip comprises a non-woven fabric sandwiched between plastic layers each having a thickness of 15 to 35 @m, said fabric having a basis weight range of 7 to 100g/m$^2$.

(9) The pressure sensitive tape of claim (1) wherein said fastening tape is formed of a self-wound tape stock roll, said roll comprising longitudinally coextensive first and second strips defined by a fold of a continuous base strip, said second strip carrying a continuous band of adhesive coatings on its upperside and a continuous band of release coatings on its underside which receives a continuous band of adhesive coatings provided on an upperside of the first strip, said base strip having a multiplicity of intermittent slit lines which extends across the whole width thereof for defining individual fastening tapes in a juxtaposed relationship.

(10) The fastening tape of Claim (9) wherein said first strip of the tape stock roll has a longitudinally-extending intermittent-slit curved line along a lateral edge thereof so as to provide the individual tape with a curvilinear terminal edge.

(11) The fastening tape of Claim (9) wherein at least one lateral edge of the continuous base strip is cut along a curved line to provide the individual tape with at least one curvilinear edge.

# FIG.1

# FIG.2

# FIG.3

# FIG.3

15  21  19  22  W

17a  18a

17  18  23

# FIG.4

15  21  19  22  23

20  17  16  18

# FIG.5

16b ] 
16a } 16
16b ]

# FIG.4

# FIG.5

# FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 168 196 (S.B. NEMETH) <br> * Column 2, lines 51-68; column 3, lines 1-47; column 4, lines 30-41; figures * | 1,9 | A 41 B 13/02 <br> C 09 J 7/04 |
| A | FR-A-2 259 551 (COLGATE PALMOLIVE CO.) <br> * Page 3, lines 31-37; page 4, lines 1-35; claims 1,6; figures * | 1,2 | |
| A | FR-A-2 485 893 (VEREINIGTE PAPIERWERKE SCHICKEDANZ & CO.) <br> * Claims; figures * | 2,3 | |
| A | EP-A-0 113 464 (UNI-CHARM CORP.) <br> * Page 10, paragraph 2; page 12, paragraph 2 * | 8 | |
| A | US-A-3 955 576 (KIMBERLY-CLARK CORP.) | | |

TECHNICAL FIELDS
SEARCHED (Int. Cl.4)

A 41 B
C 09 J

The present search report has been drawn up for all claims

| Place of search | Date of completion of the search | Examiner |
|---|---|---|
| THE HAGUE | 22-08-1988 | GARNIER F.M.A.C. |